# EUROPEAN PATENT APPLICATION

(11) **EP 3 449 964 A1**
(43) Date of publication of application: **06.03.2019**
(21) Application number: 18159910.1
(22) Date of filing: 05.03.2018
(51) Int. Cl.: A61M 11/00, A61M 15/00

(54) **NEBULIZER AND METHOD FOR OPERATING THE SAME**

(30) Priority: 04.09.2017 TW 106130103
(71) Applicant: Delta Electronics, Inc., Taoyuan City 333 (TW)
(72) Inventor: Pai, Sheng-Wen, 333 Taoyuan City (TW); Shih, Yu-Jia, 333 Taoyuan City (TW)
(74) Representative: Nederlandsch Octrooibureau

(57) **Abstract**

Present disclosure relates to a nebulizer (1) and a method for operating the same. The nebulizer includes a nebulizing circuit (100), a power providing circuit (200), a selection interface (400) and a control circuit (300). The nebulizing circuit includes a piezoelectric actuator (101). The selection interface is configured to select between a first parameter set and a second parameter set, wherein the first and the second parameter sets are set corresponding to a first solution and a second solution respectively. When the first parameter set is selected, the control circuit controls the power providing circuit to drive the nebulizing circuit with a first voltage, and the first solution is nebulized in a first particle size. When the second parameter set is selected, the control circuit controls the power providing circuit to drive the nebulizing circuit with a second voltage, and the second solution is nebulized in a second particle size.

## Description

### BACKGROUND

### Field of Invention

Present disclosure relates to a nebulizer and a method for operating the nebulizer. More particularly, present disclosure relates to a nebulizer configured to nebulize solutions according to their types and a method for operating said nebulizer.

### Description of Related Art

The power and voltage to operate existed nebulizers are usually fixed, and the users may not adjust them according to demands. However, different medicinal solutions may have different physical properties and densities. When a nebulizer is being provided to nebulize different medicinal solutions, the power and voltage provided to operate the nebulizer influences the nebulizing time, particle sizes, and bioavailability to users.

By the vibration of the actuator, medicinal solutions are nebulized through tiny holes on the nebulizer. However, when nebulizing some medicinal solutions, high power or voltage drives the piezoelectric actuator to vibrate severely, and the medicinal solutions will be nebulized into larger particle sizes. In some cases, small particles of the nebulized medicinal solutions will hit larger particles to form much larger particles of the nebulized medicinal solutions. Under mentioned circumstances, it will be difficult for the nebulized medicinal solutions to penetrate the user's lungs. On the other hand, a smaller power or voltage may drive nebulize the medicinal solutions into smaller particle sizes, but it is time consuming. Therefore, since existing nebulizers are far from ideal, improvements are required.

### SUMMARY

The disclosure provides a nebulizer, wherein the nebulizer comprises a nebulizing circuit, a power providing circuit, a selection interface, and a control circuit. The nebulizing circuit comprises a piezoelectric actuator. The power providing circuit is electrically coupled to the nebulizing circuit, and the power providing circuit is configured to selectively provide voltages to the nebulizing circuit. The selection interface is configured to select a first parameter set or a second parameter set, wherein the first parameter set is corresponding to a first solution and the second parameter set is corresponding to a second solution. The control circuit is electrically coupled to the selection interface and the power providing circuit, wherein the control circuit is configured to control the power providing circuit to provide a first voltage to the nebulizing circuit when the first parameter set is selected, and the piezoelectric actuator is driven to nebulize the first solution according to a first particle size, and wherein the control circuit is configured to control the power providing circuit to provide a second voltage to the nebulizing circuit when the second parameter set is selected, and the piezoelectric actuator is driven to nebulize the second solution according to a second particle size.

Another aspect of present disclosure is to provide a method for operating a nebulizer, wherein the nebulizer comprises a power providing circuit, a nebulizing circuit, a control circuit, and a selection interface, wherein the nebulizing circuit is electrically coupled to the power providing circuit and further comprises a piezoelectric actuator, wherein the control circuit is electrically coupled to the selection interface to control the power providing circuit. The method comprises following steps: providing a first parameter set or a second parameter set by the selection interface, wherein the first parameter set is corresponding to a first solution and the second parameter set is corresponding to a second solution; controlling the power providing circuit to provide a first voltage to the nebulizing circuit by the control circuit when the first parameter set is selected, and the piezoelectric actuator is driven to nebulize the first solution according to a first particle size; and controlling the power providing circuit to provide a second voltage to the nebulizing circuit by the control circuit when the second parameter set is selected, and the piezoelectric actuator is driven to nebulize the second solution according to a second particle size.

It is to be understood that both the foregoing general description and the following detailed description are by examples, and are intended to provide further explanation of the disclosure as claimed.

### BRIEF DESCRIPTION OF THE DRAWINGS

Present disclosure can be more fully understood by reading the following detailed description of the embodiment, with reference made to the accompanying drawings as follows:
Fig. 1 is a schematic diagram of a nebulizer according to some embodiments of the present disclosure;
Fig. 2 is a schematic diagram of a nebulizer according to some embodiments of the present disclosure;
Fig. 3 is a schematic diagram of a nebulizer according to some embodiments of the present disclosure;
Fig. 4 is a schematic diagram of a nebulizer according to some embodiments of the present disclosure;
Fig. 5 is a schematic diagram of a nebulizer according to some embodiments of the present disclosure;
Fig. 6 is a flow chart of a method for operating a nebulizer to some embodiments of the present disclosure;
Fig. 7 is a flow chart of a method for operating a nebulizer to some embodiments of the present disclosure.

### DETAILED DESCRIPTION

Reference will now be made in detail to the present embodiments of the disclosure, examples of which are illustrated in the accompanying drawings. Wherever possible, the same reference numbers are used in the drawings and the description to refer to the same or like parts.

The terms used in this specification generally have their ordinary meanings in the art and in the specific context where each term is used. The use of examples in this specification, including examples of any terms discussed herein, is illustrative only, and in no way limits the scope and meaning of the disclosure or of any exemplified term. Likewise, the present disclosure is not limited to various embodiments given in this specification.

As used herein, the terms "comprising," "including," "having," and the like are to be understood to be open-ended, i.e., to mean including but not limited to.

Reference throughout the specification to "one embodiment" or "an embodiment" means that a particular feature, structure, implementation, or characteristic described in connection with the embodiment is included in at least one embodiment of the present disclosure. Thus, uses of the phrases "in one embodiment" or "in an embodiment" in various places throughout the specification are not necessarily all referring to the same embodiment. Furthermore, the particular features, structures, implementation, or characteristics may be combined in any suitable manner in one or more embodiments.

In the following description and claims, the terms "coupled" and "connected", along with their derivatives, may be used. In particular embodiments, "connected" and "coupled" may be used to indicate that two or more elements are in direct physical or electrical contact with each other, or may also mean that two or more elements may be in indirect contact with each other. "Coupled" and "connected" may still be used to indicate that two or more elements cooperate or interact with each other.

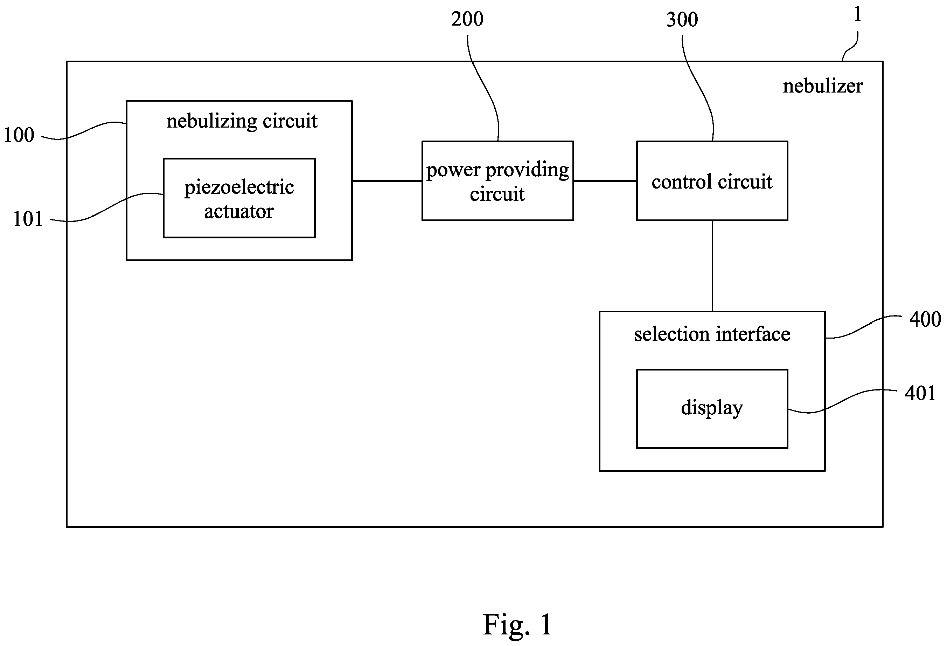

Fig. 1 is a schematic diagram of a nebulizer according to some embodiments of the present disclosure. In the embodiment, a nebulizer 1 comprises a nebulizing circuit 100, a power providing circuit 200, a control circuit 300, and a selection interface 400. The nebulizing circuit 100 includes a piezoelectric actuator 101 and is electrically coupled to the power providing circuit 200. The piezoelectric actuator 101, for example, may be a unit including capacitor, resistor and inductor, wherein when the piezoelectric actuator 101 is provided with voltages, the piezoelectric actuator 101 operates according to an operating frequency. The power providing circuit 200 is electrically coupled to the nebulizing circuit 100, wherein the power providing circuit 200 may selectively provide different voltages to the nebulizing circuit 100. The control circuit 300 is electrically coupled to the power providing circuit 200, wherein the control circuit 300 is configured to control voltages that the power providing circuit 200 provides to the nebulizing circuit 100, and to control the time that the power providing circuit 200 provides the voltages. The selection interface 400 is electrically coupled to the control circuit 300. The selection interface 400 is configured to select a first parameter set or a second parameter set, wherein the first parameter set is provided to nebulize a first solution and the second parameter set is provided to nebulize a second solution. The selection interface 400 have a display 401, wherein the display 401 is configured to display a first option corresponding to the first parameter set or a second option corresponding to the second parameter set graphically so that a user may select between them. In addition, the selection interface 400 may be configured to select among more than two parameter sets, and each parameter set is corresponding to a type of solutions. In other words, in this configuration, there may be more than two kinds of options being display on the display 401, and the user may select among them.

In the embodiment, the nebulizer 1 is configured to nebulize medicinal solutions. The first solution loaded in the nebulizer 1 may be, but not limited to, a kind of medicinal solution which contains Budesonide. The second solution loaded in the nebulizer 1 may be, but not limited to, a kind of medicinal solution which contains Bisolvon. In the embodiment, the first parameter set corresponding to the first solution includes, but not limited to, a 28V driving voltage, and the second parameter set corresponding to the second solution includes, but not limited to, a 27V driving voltage. The given voltages may be modified based on the density of the medicinal solutions so as to provide nebulized medicines with better particle sizes and/or durations.

In the embodiment, when the user selects the first option on the display 401, the selection interface 400 correspondingly selects the first parameter set as an input to the control circuit 300, and the control circuit 300 may control the power providing circuit 200 according to the first parameter set. The power providing circuit 200 may provide the first voltage (28V) to the nebulizing circuit 100, and the piezoelectric actuator 101 may be driven to operate according to a first operating frequency. When the piezoelectric actuator 101 is operating according to the first operating frequency, the first solution (medicinal solution which contains Budesonide) loaded in the nebulizer 1 may be nebulized in mist, and the first solution is being nebulized in a first particle size. In an exemplary embodiment, the first particle size is smaller than 8µm, and that particle size provides better bioavailability when the nebulized first solution reaches the user's lungs. In some embodiments, when the user selects the first option on the display 401, according to the first parameter set, the control circuit 300 controls the power providing circuit 200 to provided the first voltage to the nebulizing circuit 100 in a first duration. Therefore, the control circuit 300 may control the time that the first solution being nebulized.

In the embodiment, when the user selects the second option on the display 401, the selection interface 400 correspondingly selects the second parameter set as an input to the control circuit 300, and the control circuit 300 may control the power providing circuit 200 according to the second parameter set. The power providing circuit 200 may provide the second voltage (27V) to the nebulizing circuit 100, and the piezoelectric actuator 101 may be driven to operate according to a second operating frequency. When the piezoelectric actuator 101 is operating according to the second operating frequency, the second solution (medicinal solution which contains Bisolvon) loaded in the nebulizer 1 may be nebulized into mist, and the second solution is being nebulized in a second particle size. In an exemplary embodiment, the second particle size is smaller than 8µm, and that particle size provides better bioavailability when the nebulized second solution reaches the user's lungs. In some embodiments, when the user selects the second option on the display 401, according to the second parameter set, the control circuit 300 controls the power providing circuit 200 to provided the second voltage to the nebulizing circuit 100 in a second duration. Therefore, the control circuit 300 may control the time that the second solution being nebulized.

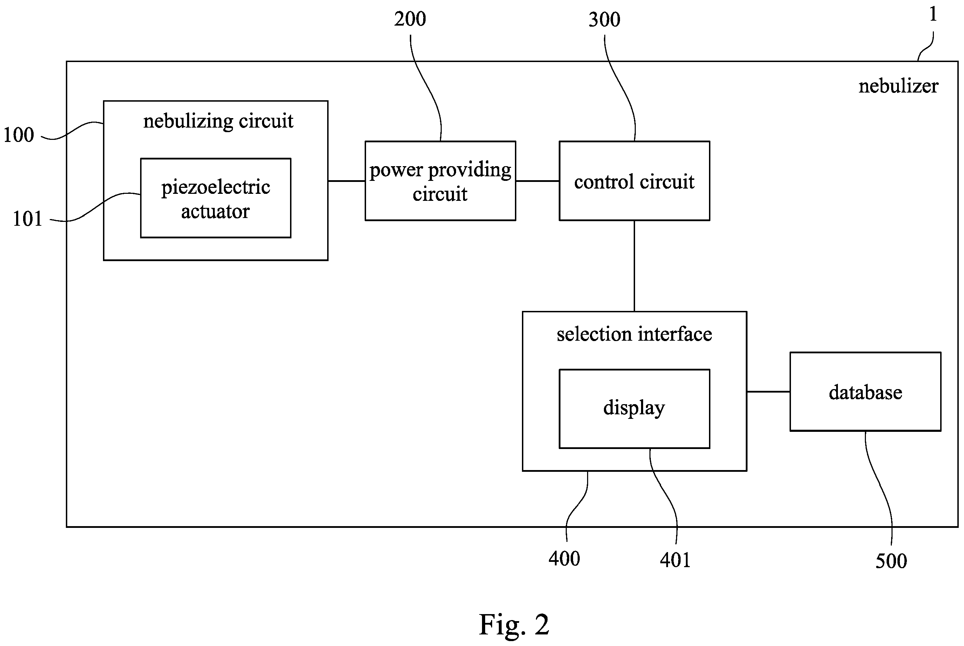

Fig. 2 is a schematic diagram of a nebulizer according to some embodiments of the present disclosure. As same as the embodiment shown in Fig. 1, the nebulizer 1 comprises the nebulizing circuit 100, the power providing circuit 200, the control circuit 300 and the selection interface 400. However, the nebulizer 1 in the embodiment further comprises a database 500. It should be understood that units labeled with same reference signs may be referenced to the embodiment of Fig. 1 and not repeated again.

In the embodiment, the database 500 is electrically coupled to the selection interface 400. The database 500 is configured to at least store the first parameter set and the second parameter set, wherein the first parameter set and the second parameter set may be accessed by the selection interface 400. The database 500 further stores a first duration and a second duration, wherein the first duration (for example, 60 seconds, according to the selected medicine, but not limited to) corresponds to the first parameter set and the second duration (for example, 90 seconds, according to the selected medicine, but not limited to) corresponds to the second parameter set. In some embodiments, the first parameter set and the second parameter set may be accessed and updated by some external devices (not shown in figure). In some embodiments, the database 500 stores a medicinal list, in which the medicinal list includes information regarding to multiple medicinal solutions, and each of the medicinal solutions corresponds to a suitable parameter set (includes a suitable voltage) and a suitable nebulizing duration. Based on demands, the selection interface 400 may access suitable parameter set corresponding to different medicinal solutions from the database 500, and the nebulizer 1 may nebulize specific medicinal solutions accordingly. As such, the nebulizer 1 may administrate dosage and particle sizes in a suitable manner when nebulizing medicinal solutions, therefore better bioavailability may be achieved.

In the embodiment, the nebulizer 1 is configured to nebulize medicinal solutions. The first solution loaded in the nebulizer 1 may be, but not limited to, a kind of medicinal solution contains Atrovent. The second solution loaded in the nebulizer 1 may be, but not limited to, a kind of medicinal solution which contains Combivent. In the embodiment, the first parameter set corresponding to the first solution includes, but not limited to, a 27V driving voltage, and the second parameter set corresponding to the second solution includes, but not limited to, a 27V driving voltage. The given voltages may be modified based on the density of the medicinal solutions so as to provide nebulized medicinal solutions with better particle sizes and/or durations.

In the embodiment, when the user selects the first option on the display 401, the selection interface 400 may access the first parameter set and the first duration from the database 500, wherein the first parameter set and the first duration are input to the control circuit 300. Therefore, the control circuit 300 may control the power providing circuit 200 according to the first parameter set in the first duration (60 seconds). According to the first parameter set, the power providing circuit 200 may provide the first voltage (27V) to the nebulizing circuit 100, and the piezoelectric actuator 101 may be driven to operate according to a first operating frequency. When the piezoelectric actuator 101 is operating according to the first operating frequency, the first solution (a medicinal solution which contains Atrovent) loaded in the nebulizer 1 may be nebulized in mist, and the first solution is being nebulized in a first particle size. In an exemplary embodiment, the first particle size is smaller than 8µm, and that particle size provides better bioavailability when the nebulized first solution reaches the user's lungs. When 60 seconds (the first duration) is reached, the control circuit 300 disables the power providing circuit 200 to provide the first voltage to the nebulizing circuit 100, therefore the nebulizing process of the first solution is stopped.

In the embodiment, when the user selects the second option on the display 401, the selection interface 400 may access the second parameter set and the second duration from the database 500, wherein the second parameter set and the second duration are input to the control circuit 300. Therefore, the control circuit 300 may control the power providing circuit 200 according to the second parameter set in the second duration (90 seconds). According to the second parameter set, the power providing circuit 200 may provide the second voltage (27V) to the nebulizing circuit 100, and the piezoelectric actuator 101 may be driven to operate according to a second operating frequency. When the piezoelectric actuator 101 is operating according to the second operating frequency, the second solution (a medicinal solution which contains Combivent) loaded in the nebulizer 1 may be nebulized in mist, and the second solution is being nebulized in a second particle size. In an exemplary embodiment, the second particle size is smaller than 8µm, and that particle size provides better bioavailability when the nebulized second solution reaches the user's lungs. When 90 seconds (the second duration) is reached, the control circuit 300 disables the power providing circuit 200 to provide the second voltage to the nebulizing circuit 100, therefore the nebulizing process of the second solution is stopped.

It should be understood, despite the database 500 is comprised by the nebulizer 1 in aforementioned embodiment, the configuration of the database is not limited thereto. For instance, in some embodiments, the database may be configured in some external devices or servers. However, as long as the database is in communication with the nebulizer 1 of present disclosure, the selection interface 400 may access parameter sets and durations correspondingly.

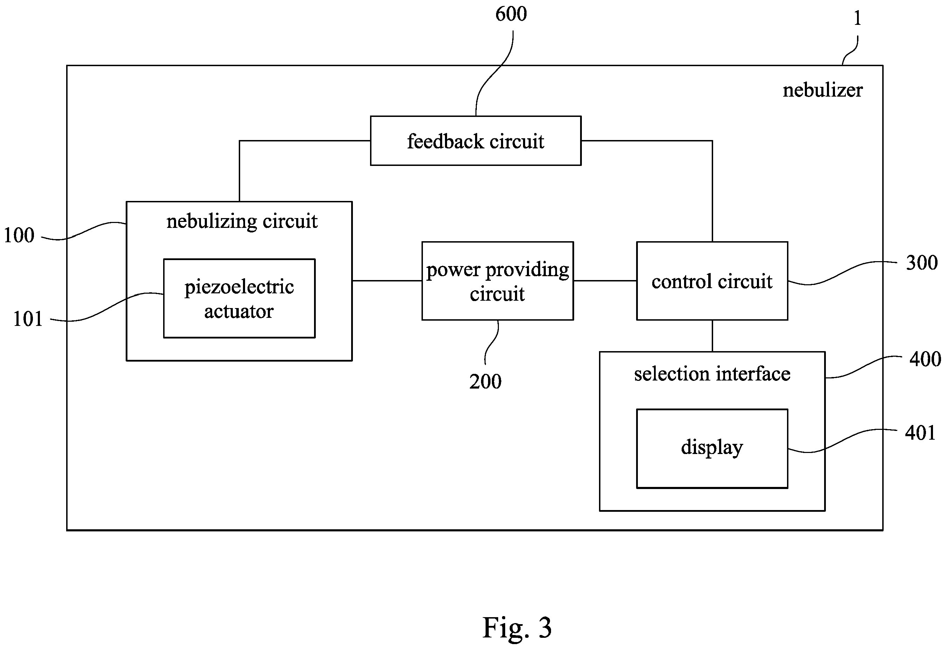

Fig. 3 is a schematic diagram of a nebulizer according to some embodiments of the present disclosure. As same as the embodiment shown in Fig. 1, the nebulizer 1 comprises the nebulizing circuit 100, the power providing circuit 200, the control circuit 300 and the selection interface 400. However, the nebulizer 1 in the embodiment further comprises a feedback circuit 600. It should be understood that units labeled with same reference signs may be referenced to the embodiment of Fig. 1, and not repeated again.

In the embodiment, the feedback circuit 600 is electrically connected between the nebulizing circuit 100 and the control circuit 300. The feedback circuit 600 may comprise a sampling circuit (not shown in figure) and a filtering circuit (not shown in figure). The sampling circuit is configured to sample signals generated when the piezoelectric actuator 101 is working in the operating frequency, wherein the signals may be, but not limited to, harmonic signals and pulse signals. The filtering circuit is electrically coupled to the sampling circuit, wherein the filtering circuit is configured to obtain specific frequencies from the signals sampled by the sampling circuit. As such, the feedback circuit 600 may send the filtered signals corresponding to the operating frequency of the piezoelectric actuator 101 to the control circuit 300.

In the embodiment, the nebulizer 1 is configured to nebulize medicinal solutions. The first solution loaded in the nebulizer 1 may be, but not limited to, a kind of medicinal solution which contains Budesonide. The second solution loaded in the nebulizer 1 may be, but not limited to, a kind of medicinal solution which contains Bisolvon. In the embodiment, the first parameter set corresponding to the first solution includes, but not limited to, a 28V driving voltage, and the second parameter set corresponding to the second solution includes, but not limited to, a 27V driving voltage. The given voltages may be modified based on the density of the medicinal solutions so as to provide nebulized medicines with better particle sizes and/or durations.

In the embodiment, when the user selects the first option on the display 401, the selection interface 400 may select the first parameter set as an input to the control circuit 300. According to the first parameter set, the control circuit 300 may control the power providing circuit 200 to provide the first voltage (28V) to the nebulizing circuit 100, and the piezoelectric actuator 101 may be driven to operate according to a first operating frequency. When the piezoelectric actuator 101 is operating according to the first operating frequency, the first solution (medicine medicinal solution which contains Budesonide) loaded in the nebulizer 1 may be nebulized in mist, and the first solution is being nebulized in a first particle size. In an exemplary embodiment, the first particle size is smaller than 8µm. When the piezoelectric actuator 101 is operating, the feedback circuit 600 may obtain signals generated by the operation of the piezoelectric actuator 101 and send the signals to the control circuit 300. When the control circuit 300 determines the signals obtained by the feedback circuit 600 reflects that the piezoelectric actuator 101 is not operating in the first frequency or voltage, the control circuit 300 may disable the power providing circuit 200 to provide the first voltage to the nebulizing circuit 100, therefore the nebulizing process of the first solution is stopped. As mentioned, the configuration of the feedback circuit 600 can be considered as an error monitoring circuit, it may stop the nebulizing process when the user selects incorrect parameter sets to nebulize medicinal solutions. In some embodiments, the control circuit 300 may further send an alarm to the selection interface 400, and the user may read said circumstances on the display 401.

In the embodiment, when the user selects the second option on the display 401, the selection interface 400 may select the second parameter set as an input to the control circuit 300. According to the second parameter set, the control circuit 300 may control the power providing circuit 200 to provide the second voltage (27V) to the nebulizing circuit 100, and the piezoelectric actuator 101 may be driven to operate according to a second operating frequency. When the piezoelectric actuator 101 is operating according to the second operating frequency, the second solution (a medicinal solution which contains Bisolvon) loaded in the nebulizer 1 may be nebulized in mist, and the second solution is being nebulized in a second particle size. In an exemplary embodiment, the second particle size is smaller than 8µm. When the piezoelectric actuator 101 is operating, the feedback circuit 600 may obtain signals generated by the operation of the piezoelectric actuator 101 and send the signals to the control circuit 300. When the control circuit 300 determines the signals obtained by the feedback circuit 600 reflects that the piezoelectric actuator 101 is not operating in the second frequency or voltage, the control circuit 300 may disable the power providing circuit 200 to provide the second voltage to the nebulizing circuit 100, therefore the nebulizing process of the second solution is stopped. The configuration of the feedback circuit 600 can be considered as an error monitoring circuit, it may stop the nebulizing process when the user selects incorrect parameter sets to nebulize medicinal solutions. In some embodiments, the control circuit 300 may further send an alarm to the selection interface 400, and the user may read said circumstances on the display 401.

In some embodiments, when the control circuit 300 determines that the signals obtained by the feedback circuit 600 reflects that the piezoelectric actuator 101 is not operating in the first frequency, the second frequency, the first voltage or the second voltage normally, the control circuit 300 may access a database to search if the signals obtained by the feedback circuit 600 is closed to the first frequency, the second frequency, the first voltage or the second voltage and the control circuit 300 may automatically apply corresponding parameter sets to correct the nebulizing process.

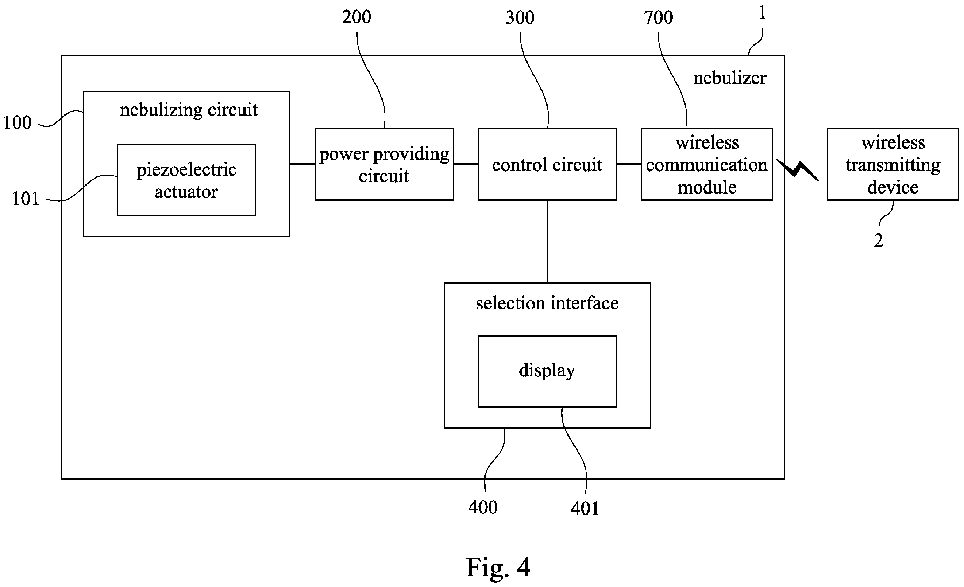

Fig. 4 is a schematic diagram of a nebulizer according to some embodiments of the present disclosure. As same as the embodiment shown in Fig. 1, the nebulizer 1 comprises the nebulizing circuit 100, the power providing circuit 200, the control circuit 300 and the selection interface 400. However, the nebulizer 1 in the embodiment further comprises a wireless communication module 700. It should be understood that units labeled with same reference signs may be referenced to the embodiment of Fig. 1, and not repeated again.

In the embodiment, the wireless communication module 700 is electrically coupled to the control circuit 300, and the wireless communication module 700 is in communication with a wireless transmitting device 2. The wireless communication module 700 may receive a control command from the wireless transmitting device 2, wherein the control command is provided to select between the first parameter set and the second parameter set. It is to say, in aforementioned embodiments, the user may select the first option or the second option on the display 401 of the selection interface 400, and the control circuit 300 may control the power providing circuit 200 according to the first parameter set or the second parameter set correspondingly. However, in this embodiment, the user may send the control command via the wireless transmitting device, and the control circuit 300 may control the power providing circuit 200 according to the first parameter set or the second parameter set being selected. As such, an alternative to control the nebulizer 1 of present disclosure is provided. Even though the user may not reach the selection interface 400 of the nebulizer 1, he may still control the nebulizer 1 by the control command, and the nebulizer 1 may administrate dosage and particle sizes in a suitable manner when nebulizing medicinal solutions.

In the embodiment, when the wireless communication module 700 receives the control command from the wireless transmitting device 2, the control circuit 300 may select the first parameter set or the second parameter set according to the control command, and the power providing circuit 200 is controlled to provide the first or second voltage to the nebulizing circuit 100 accordingly. The process after the voltages being provided to the nebulizing circuit 100 can be referenced to the aforementioned embodiments, and not repeated again.

It should be understood, in some embodiments of present disclosure, the control command sending from the wireless transmitting device 2 further includes information about modifying the first voltage and the first duration in the first parameter set, or includes information about modifying the second voltage and the second duration in the second parameter set. Moreover, the control command may further include information about modifying voltages and durations in other parameter sets. It is to say, the control command sending from the wireless transmitting device 2 can also be used to update or add more parameters into the parameter sets. For example, in a combination of this embodiment and the embodiment of Fig. 2, when the wireless communication module 700 receives the control command from the wireless transmitting device 2, the control circuit 300 may access the database 500 to modify the voltages or durations stored in the first parameter set or the second parameter set according to the control command. Furthermore, according to the control command, the control circuit 300 may also access the database 500 to add a new third parameter set corresponding to other medicinal solution. As such, the embodiment provides an approach to modify voltages and durations for the nebulizer 1 to nebulize the medicinal solutions.

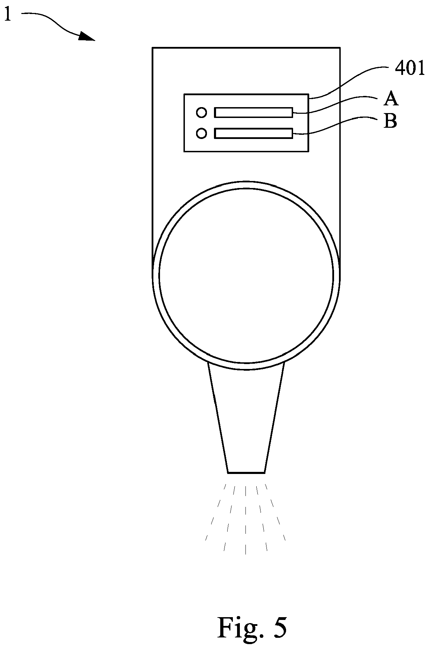

Fig. 5 is a schematic diagram of a nebulizer according to some embodiments of the present disclosure. The appearance of a nebulizer 1 is shown from above in the figure. However, only the appearance of the nebulizer 1 is shown in the figure, please referring to the embodiments shown in the Fig. 1 to Fig. 4 regarding to the internal circuit structure of the nebulizer 1. It can be seen in Fig. 5 that the display 401 is settled on the top of the nebulizer 1, in which the display 401 may be a liquid crystal display (LCD). The display 401 shown in Fig. 5 presents the appearance of the display 401 described in the embodiments of Fig. 1 to Fig. 4. A first option A and a second option B are shown on the display 401, the user may touch the display 401 to select the first option A or the second option B. Once the first option A or the second option B is selected, the selection interface (not shown in Fig. 5) may select a first parameter set corresponding to the first option A or select a second parameter set corresponding to the second option B as an input to the control circuit (not shown in Fig. 5), and therefore the first solution or the second solution may be nebulized.

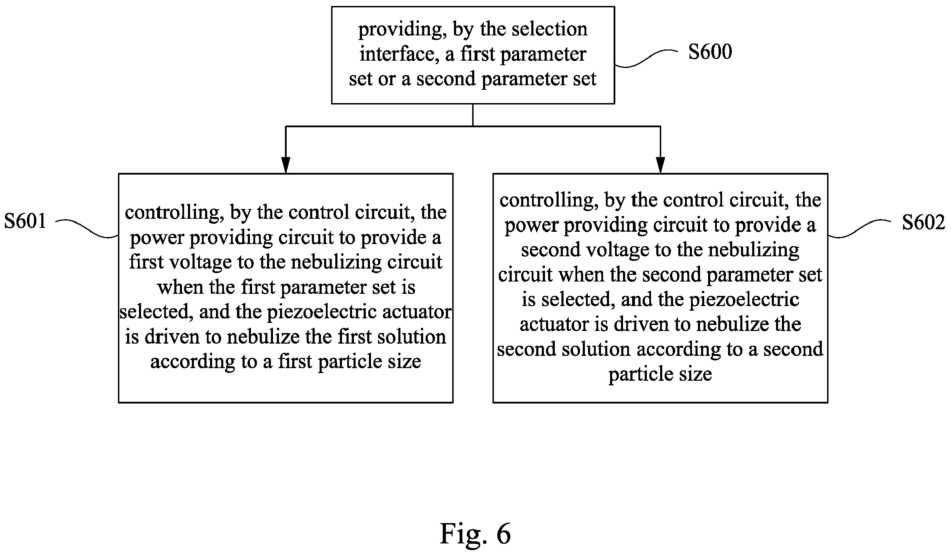

Fig. 6 is a flow chart of a method for operating a nebulizer according to some embodiments of the present disclosure. In the embodiment, the method is provided to operate a nebulizer as shown in the embodiments of Fig. 1 to Fig. 5. For example, as shown on Fig. 1, the nebulizer 1 comprises the power providing circuit 200 and the nebulizing circuit 100, wherein the nebulizing circuit 100 is electrically coupled to the power providing circuit 200 and further comprises the piezoelectric actuator 101. The nebulizer 1 further comprises the control circuit 300 and the selection interface 400, wherein the control circuit 300 is electrically coupled to the selection interface 400 to control the power providing circuit 200. In the embodiment, the steps of the method will be listed and explained in detail in following segments.

Step S600: providing, by the selection interface, a first parameter set or a second parameter set, wherein the first parameter set is corresponding to a first solution and the second parameter set is corresponding to a second solution. As shown in Fig. 1, the selection interface 400 may comprise a display, in which the display is configured to display options corresponding to the first parameter set or the second parameter set in graphics. For example, as shown in Fig. 1 and Fig. 5, the display 401 of the nebulizer shown in Fig. 5 is configured to display a first option A corresponding to the first parameter set or a second option B corresponding to the second parameter set. The user may touch the display 401 to select the first option A or the second option B. Once the first option A or the second option B is selected, the selection interface 400 shown in Fig. 1 may select the first parameter set or the second parameter set as an input to the control circuit 300 correspondingly.

Step S601: controlling, by the control circuit, the power providing circuit to provide a first voltage to the nebulizing circuit when the first parameter set is selected, and the piezoelectric actuator is driven to nebulize the first solution according to a first particle size. As shown in Fig. 1 and Fig. 5, when the user selects the first option A on the display 401, the selection interface 400 may select the first parameter set corresponding to the first option A as an input to the control circuit 300, and the control circuit 300 may control the power providing circuit 200 to provide the first voltage to the nebulizing circuit 100. The piezoelectric actuator 101 may be driven to nebulize the first solution in the first particle size. Since the first particle size is set corresponding to the first solution, it can make the nebulized first solution more effective. In some embodiments of present disclosure, as shown in Fig.2 and Fig.5, when the user selects the first option A on the display 401, the selection interface 400 shown in Fig. 2 may access the database 500 for the first parameter set and further input the first parameter set to the control circuit 300.

Step S602: controlling, by the control circuit, the power providing circuit to provide a second voltage to the nebulizing circuit when the second parameter set is selected, and the piezoelectric actuator is driven to nebulize the second solution according to a second particle size. As shown in Fig. 1 and Fig. 5, when the user selects the second option B on the display 401, the selection interface 400 may select the second parameter set corresponding to the second option B as an input to the control circuit 300, and the control circuit 300 may control the power providing circuit 200 to provide the second voltage to the nebulizing circuit 100. The piezoelectric actuator 101 may be driven to nebulize the second solution in the second particle size. Since the second particle size is set corresponding to the second solution, it can make the nebulized second solution more effective. In some embodiments of present disclosure, as shown in Fig.2 and Fig.5, when the user selects the second option B on the display 401, the selection interface 400 shown in Fig. 2 may access the database 500 for the second parameter set and further input the second parameter set to the control circuit 300.

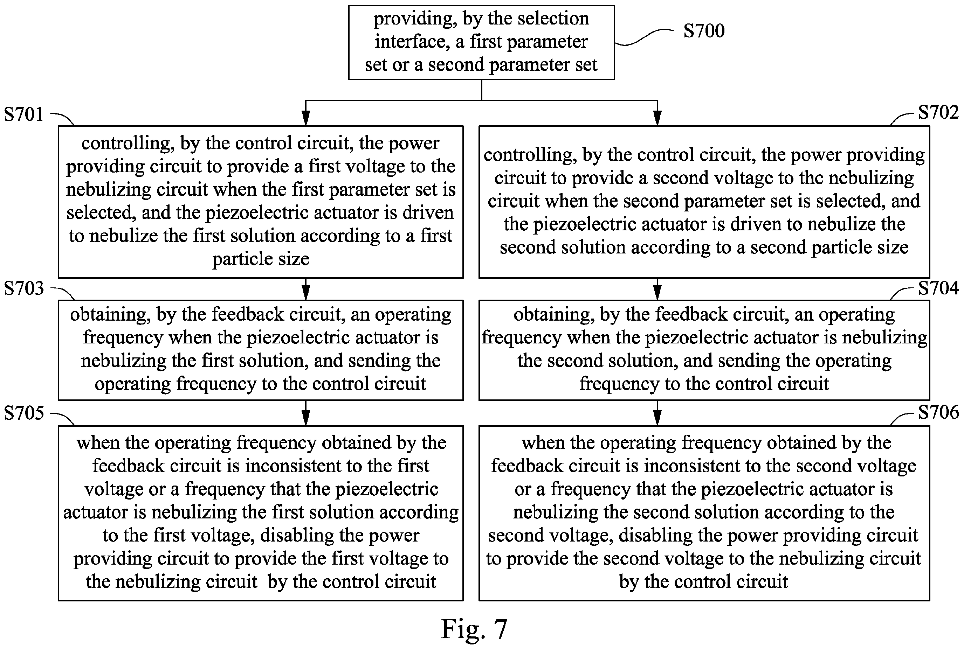

Fig. 7 is a flow chart of a method for operating a nebulizer according to some embodiments of the present disclosure. In the embodiment, the method is provided to operate a nebulizer as shown in the embodiments of Fig. 3 to Fig. 5. For example, as shown on Fig. 3, the nebulizer 1 comprises the power providing circuit 200 and the nebulizing circuit 100, wherein the nebulizing circuit 100 is electrically coupled to the power providing circuit 200 and further comprises the piezoelectric actuator 101. The nebulizer 1 further comprises the control circuit 300 and the selection interface 400, wherein the control circuit 300 is electrically coupled to the selection interface 400 to control the power providing circuit 200. Moreover, nebulizer 1 further comprises the feedback circuit 600, in which the feedback circuit 600 is electrically coupled between the nebulizing circuit 100 and the control circuit 300. In the embodiment, the steps of the method will be listed and explained in detail in following segments.

Step S700: providing, by the selection interface, a first parameter set or a second parameter set, wherein the first parameter set is corresponding to a first solution and the second parameter set is corresponding to a second solution. As shown in Fig. 3, the selection interface 400 may comprise a display 401, in which the display 401 is configured to display options corresponding to the first parameter set or the second parameter set in graphics. For example, as shown in Fig. 3 and Fig. 5, the display 401 of the nebulizer shown in Fig. 5 is configured to display a first option A corresponding to the first parameter set or a second option B corresponding to the second parameter set. The user may touch the display 401 to select the first option A or the second option B. Once the first option A or the second option B is selected, the selection interface 400 shown in Fig. 1 may select the first parameter set or the second parameter set as an input to the control circuit 300 correspondingly.

Step S701: follows step S700; controlling, by the control circuit, the power providing circuit to provide a first voltage to the nebulizing circuit when the first parameter set is selected, and the piezoelectric actuator is driven to nebulize the first solution according to a first particle size. As shown in Fig. 3 and Fig. 5, when the user selects the first option A on the display 401, the selection interface 400 may select the first parameter set corresponding to the first option A as an input to the control circuit 300, and the control circuit 300 may control the power providing circuit 200 to provide the first voltage to the nebulizing circuit 100. The piezoelectric actuator 101 may be driven to nebulize the first solution in the first particle size. Since the first particle size is set corresponding to the first solution, it can make the nebulized first solution more effective.

Step S702: follows step S700; controlling, by the control circuit, the power providing circuit to provide a second voltage to the nebulizing circuit when the second parameter set is selected, and the piezoelectric actuator is driven to nebulize the second solution according to a second particle size. As shown in Fig. 3 and Fig. 5, when the user selects the second option B on the display 401, the selection interface 400 may select the second parameter set corresponding to the second option B as an input to the control circuit 300, and the control circuit 300 may control the power providing circuit 200 to provide the second voltage to the nebulizing circuit 100. The piezoelectric actuator 101 may be driven to nebulize the second solution in the second particle size. Since the second particle size is set corresponding to the second solution, it can make the nebulized second solution more effective.

Step S703: follows step S701; obtaining, by the feedback circuit 600, an operating frequency when the piezoelectric actuator 101 is nebulizing the first solution, and sending the operating frequency to the control circuit 300. As shown in Fig. 3, when the first parameter set is input to the control circuit 300, the control circuit 300 controls the power providing circuit 200 to provided the first voltage to the nebulizing circuit 100, and the piezoelectric actuator 101 is driven to nebulize the first solution in the first particle size. When the piezoelectric actuator 101 is operating, the feedback circuit 600 keeps sampling the signals corresponding to the operation frequency of the piezoelectric actuator 101. The feedback circuit 600 may filter the signals and send the filtered signals to the control circuit 300.

Step S704: follows step S702; obtaining, by the feedback circuit 600, an operating frequency when the piezoelectric actuator 101 is nebulizing the second solution, and sending the operating frequency to the control circuit 300. As shown in Fig. 3, when the second parameter set is input to the control circuit 300, the control circuit 300 controls the power providing circuit 200 to provided the second voltage to the nebulizing circuit 100, and the piezoelectric actuator 101 is driven to nebulize the second solution in the second particle size. When the piezoelectric actuator 101 is operating, the feedback circuit 600 keeps sampling the signals corresponding to the operation frequency of the piezoelectric actuator 101. The feedback circuit 600 may filter the signals and send the filtered signals to the control circuit 300.

Step S705: follows step S703; when the operating frequency obtained by the feedback circuit 600 is inconsistent to the first voltage or a frequency that the piezoelectric actuator 101 is nebulizing the first solution according to the first voltage, disabling the power providing circuit 200 to provide the first voltage to the nebulizing circuit 100 by the control circuit 300. As shown in Fig. 3, the feedback circuit 600 keeps sampling the signals corresponding to the operation frequency of the piezoelectric actuator 101, and the feedback circuit 600 further filters the signals and send the filtered signals to the control circuit 300. The control circuit 300 may receive and compare the filtered signals with a specific frequency, wherein the specific frequency indicates that the piezoelectric actuator 101 is driven by the first voltage and operates in the first frequency. If the control circuit 300 determines the signals obtained by the feedback circuit 600 reflects that the piezoelectric actuator 101 is not operating in the first frequency or voltage, the control circuit 300 may disable the power providing circuit 200 to provide the first voltage to the nebulizing circuit 100. In some embodiments, the control circuit 300 may further send an alarm to the selection interface 400, and the user may read said circumstances on the display 401.

Step S706: follows step S704; when the operating frequency obtained by the feedback circuit 600 is inconsistent to the second voltage or a frequency that the piezoelectric actuator 101 is nebulizing the second solution according to the second voltage, disabling the power providing circuit 200 to provide the second voltage to the nebulizing circuit 100 by the control circuit 300. As shown in Fig. 3, the feedback circuit 600 keeps sampling the signals corresponding to the operation frequency of the piezoelectric actuator 101, and the feedback circuit 600 further filters the signals and send the filtered signals to the control circuit 300. The control circuit 300 may receive and compare the filtered signals with a specific frequency, wherein the specific frequency indicates that the piezoelectric actuator 101 is driven by the second voltage and operates in the second frequency. If the control circuit 300 determines the signals obtained by the feedback circuit 600 reflects that the piezoelectric actuator 101 is not operating in the second frequency or voltage, the control circuit 300 may disable the power providing circuit 200 to provide the second voltage to the nebulizing circuit 100. In some embodiments, the control circuit 300 may further send an alarm to the selection interface 400, and the user may read said circumstances on the display 401.

From abovementioned embodiments, application of present disclosure brings advantages as follows. Some embodiments of present disclosure provides a nebulizer and a method to operate such nebulizer, wherein the provided nebulizer may nebulize several types of solutions according to different particle sizes, which is unachievable to the prior arts. Some embodiments of present disclosure also provide a nebulizer and a method to operate such nebulizer, wherein the provided nebulizer may nebulize several types of solutions in several durations so as to improve the punctuality of the treatments. Some embodiments of present disclosure provides a nebulizer, wherein the provided nebulizer may feedback the operating frequencies of itself to determine if the user selects incorrect parameter sets to operate the nebulizer.

Although the present disclosure has been described in considerable detail with reference to certain embodiments thereof, other embodiments are possible. Therefore, the spirit and scope of the appended claims should not be limited to the description of the embodiments contained herein.

## Claims

1. A nebulizer (1), comprising:
a nebulizing circuit (100) comprising a piezoelectric actuator (101);
a power providing circuit (200) electrically coupled to the nebulizing circuit (100), and configured to selectively provide voltages to the nebulizing circuit (100);
a selection interface (400) configured to select a first parameter set or a second parameter set, wherein the first parameter set is corresponding to a first solution and the second parameter set is corresponding to a second solution; and
a control circuit (300) electrically coupled to the selection interface (400) and the power providing circuit (200), wherein the control circuit (300) is configured to control the power providing circuit (200) to provide a first voltage to the nebulizing circuit (100) when the first parameter set is selected, and the piezoelectric actuator (101) is driven to nebulize the first solution according to a first particle size, and wherein the control circuit (300) is configured to control the power providing circuit (200) to provide a second voltage to the nebulizing circuit (100) when the second parameter set is selected, and the piezoelectric actuator (101) is driven to nebulize the second solution according to a second particle size.

2. The nebulizer of claim 1, wherein the selection interface (400) comprises a display (401), in which the display (401) is configured to display a first option corresponding to the first parameter set or a second option corresponding to the second parameter set in graphics.

3. The nebulizer of claim 1, wherein the selection interface (400) is in communication with a database (500), in which the database (500) stores the first parameter set or the second parameter set, wherein the first parameter set or the second parameter set are accessed by the selection interface (400).

4. The nebulizer of claim 1, wherein when the first parameter set is selected, the control circuit (300) controls the power providing circuit (200) to provide the first voltage to the nebulizing circuit (100) in a first duration, wherein when the second parameter set is selected, the control circuit (300) controls the power providing circuit (200) to provide the second voltage to the nebulizing circuit (100) in a second duration.

5. The nebulizer of claim 1, further comprising:
a wireless communication module (700) electrically coupled to the control circuit (300), and configured to receive a control command from a wireless transmitting device (2), wherein the control command is provided to select the first parameter set or the second parameter set.

6. The nebulizer of claim 5, wherein the control command is further provided to modify the first voltage and the first duration corresponding to the first parameter set or to modify the second voltage and the second duration corresponding to the second parameter set.

7. The nebulizer of claim 1, further comprising:
a feedback circuit (600) electrically coupled to the nebulizing circuit (100) and the control circuit (300), and configured to obtain an operating frequency when the piezoelectric actuator (101) is nebulizing the first solution or the second solution and send the operating frequency to the control circuit (300), wherein when the operating frequency obtained by the feedback circuit is inconsistent to a frequency that the piezoelectric actuator (101) is nebulizing the first solution according to the first voltage or is inconsistent to a frequency that the piezoelectric actuator (101) is nebulizing the second solution according to the second voltage, the control circuit (300) disables the power providing circuit (200) to provide the first voltage or the second voltage to the nebulizing circuit (100).

8. A method to operate a nebulizer (1), wherein the nebulizer (1) comprises a power providing circuit (200), a nebulizing circuit (100), a control circuit (300), and a selection interface (400), wherein the nebulizing circuit (100) is electrically coupled to the power providing circuit (200) and further comprises a piezoelectric actuator (101), wherein the control circuit (300) is electrically coupled to the selection interface (400) to control the power providing circuit (200), wherein the method comprises:
providing, by the selection interface (400), a first parameter set or a second parameter set, wherein the first parameter set is corresponding to a first solution and the second parameter set is corresponding to a second solution;
controlling, by the control circuit (300), the power providing circuit (200) to provide a first voltage to the nebulizing circuit (100) when the first parameter set is selected, and the piezoelectric actuator (101) is driven to nebulize the first solution according to a first particle size; and
controlling, by the control circuit (300), the power providing circuit (200) to provide a second voltage to the nebulizing circuit (100) when the second parameter set is selected, and the piezoelectric actuator (101) is driven to nebulize the second solution according to a second particle size.

9. The method of claim 8, wherein the selection interface (400) comprises a display (401), in which the display (401) is configured to display a first option corresponding to the first parameter set or a second option corresponding to the second parameter set in graphics.

10. The method of claim 8, wherein the selection interface (400) of the nebulizer (1) is in communication with a database (500), in which the database (500) stores the first parameter set or the second parameter set, wherein the method further comprises:
accessing, by the selection interface (400), the first parameter set or the second parameter set from the database (500).

11. The method of claim 8, wherein when the first parameter set is selected, the control circuit (300) controls the power providing circuit (200) to provide the first voltage to the nebulizing circuit (100 in a first duration, wherein when the second parameter set is selected, the control circuit (300) controls the power providing circuit (200) to provide the second voltage to the nebulizing circuit (100) in a second duration.

12. The method of claim 11, wherein the nebulizer further comprises a wireless communication module (700), in which the wireless communication module (700) is electrically coupled to the control circuit (300), wherein the method further comprises:
receiving, by the wireless communication module (700), a control command from a wireless transmitting device (2), wherein the control command is provided to select the first parameter set or the second parameter set.

13. The method of claim 12, further comprising:
modifying, by the control circuit, the first voltage and the first duration corresponding to the first parameter set or the second voltage and the second duration corresponding to the second parameter set.

14. The method of claim 8, wherein the nebulizer further comprises a feedback circuit (600), in which the feedback circuit (600) is electrically coupled to the nebulizing circuit (100) and the control circuit (300), wherein the method further comprises:
obtaining, by the feedback circuit (600), an operating frequency when the piezoelectric actuator (101) is nebulizing the first solution or the second solution, and sending the operating frequency to the control circuit (300);
when the operating frequency obtained by the feedback circuit (600) is inconsistent to a frequency that the piezoelectric actuator (101) is nebulizing the first solution according to the first voltage, disabling the power providing circuit (200) to provide the first voltage to the nebulizing circuit (100) by the control circuit (300); and
when the operating frequency obtained by the feedback circuit (600) is inconsistent to a frequency that the piezoelectric actuator (101) is nebulizing the second solution according to the second voltage, disabling the power providing circuit (200) to provide the second voltage to the nebulizing circuit (100) by the control circuit (300).
